# EUROPEAN PATENT APPLICATION

(11) **EP 3 281 632 A1**
(43) Date of publication of application: **14.02.2018**
(21) Application number: 16183930.3
(22) Date of filing: 12.08.2016
(51) Int. Cl.: A61K 33/00, A61P 9/10, A61P 25/00

(54) **ARGON FOR USE IN THE PREVENTION OF BOTH CARDIAC AND CEREBRAL INJURY INDUCED BY ISCHEMIA/REPERFUSION FOLLOWING CARDIAC ARREST**

(71) Applicant: Societa'Italiana Acetilene & Derivati S.I.A.D. S.p.A. in breve S.I.A.D. S.p.A., 24126 Bergamo (IT)
(72) Inventor: BISSOLOTTI, Giorgio, 24100 BERGAMO (IT); RISTAGNO, Giuseppe, 20156 MILANO (IT); LATINI, Roberto, 20156 MILANO (IT); WIK, Lars, 20156 MILANO (IT)
(74) Representative: Bianchetti Bracco Minoja S.r.l.

(57) **Abstract**

The invention concerns the use of argon in the prevention of both cardiac and cerebral injury induced by ischemia/reperfusion following cardiac arrest, argon or mixtures thereof with oxygen or other inert gases being administered during cardiopulmonary resuscitation.

## Description

The invention concerns the use of argon in the prevention of both cardiac and cerebral injury induced by ischemia/reperfusion following cardiac arrest, argon or mixtures thereof with oxygen or other inert gases being administered during cardiopulmonary resuscitation.

### Background of the invention

Cardiac arrest represents a dramatic clinical event that can occur suddenly and often without premonitory signs. This condition is characterized by sudden loss of consciousness caused by the lack of cerebral blood flow, which occurs when the heart ceases to pump. Indeed, it represents a leading cause of death in the western world, with as many as 350.000 to 700.000 people in the United States, Canada and Europe sustaining cardiac arrest each year (*Lippert 2010).* Despite an initially successful return to spontaneous circulation (ROSC), prognosis of cardiac arrest remains poor, with the majority of resuscitated patients dying early due to post-cardiac arrest syndrome (*Nolan 2008).* Most prominent are post-resuscitation cardiac failure, ischemic brain damage, and the systemic inflammation related to whole-body ischemia and reperfusion. Severe heart contractile failure has been implicated as one of the most important mechanism accounting for the early fatal outcome (*Nolan 2008, Laurent 2002).* Long term morbidity and mortality after successful CPR, instead, largely depend on recovery of neurologic function (*Nolan 2008, Sandroni 2013).* As many as 30% of survivors of cardiac arrest, in fact, manifest permanent brain damage and in some instances only 2-12% of resuscitated patients have been discharged from the hospital without neurological impairments (*Brain Resuscitation Clinical Trial I 1986, Olasveengen 2009, Peberdy 2010).* Finally, the systemic inflammatory response observed after cardiopulmonary resuscitation (CPR) brings evident similarities with sepsis and septic shock, such to be generally described as a "sepsis-like" syndrome (*Adrie 2002).* Indeed, the role of inflammation in the progression towards worse circulatory shock, multiple organ dysfunction syndrome (MODS), and poor outcome of resuscitated patients is under research (*Ristagno 2013).*

Novel therapeutic approaches have been conceived and tested in the last decade, including volatile anaesthetics and noble gases, to allow for cerebral and myocardial preservation after cardiac arrest (*Fries 2008 e 2012).* Although noble gases are traditionally believed to be 'inert', these monoatomic colorless and odorless agents are indeed capable to interact with amino-acids in the active sites of several enzymes and receptors, producing biological effects (*Jawad 2009).*

The biological activity of argon was first described by deep see divers in the 1930s. Under hyperbaric conditions it has a narcotic effect but there are no anesthetic properties under normobaric conditions. Based on this argon is mostly used as an inert shielding gas in many aspects in industry processes and in medical equipment.

### Neuroprotective effects of argon have been disclosed in a number of papers.

For example, neuroprotective effects of different argon concentrations, from 25 to 95%, have shown to protect neuronal cell cultures and hippocampal slice cultures from rodents subjected to either oxygen-glucose deprivation or focal trauma (*Jawad 2009, Loetscher 2009, Yarin 2005).*

In *in vivo* studies, neuronal insult has been induced in different ways such as middle cerebral artery occlusion, intrastriatal injection, traumatic head injury, hypoxic-ischaemia, and as cardiac arrest. Administration of argon in all these situations has shown to have a neuroprotective effect through a significant reduction in infarct volume and brain damage (*Ryang 2011, David 2012, Broad 2016, Brücken 2013-2015, Zhuang 2012),* and on neurological dysfunction (*Brücken 2013, Ristagno 2014).* In addition, Ryang et al and Ristagno et al. demonstrated that normal physiological parameters were not disturbed by inhalation of argon up to 70% (*Ryang 2011, Ristagno 2014).*

The neuroprotective effects of argon have also been reported in rats resuscitated after cardiac arrest (*Brücken 2013, 2014, 2015).* In that model, post resuscitation ventilation with 70% argon showed significant improvements in neurological function and reductions in histopathological brain damage, in comparison to control ventilation. Indeed, control rats had severe neurological dysfunction, while argon-treated animals showed significant improvements in the neurological deficit score at all time points. This was paralleled to a significant reduction in the neuronal injury as shown by histology.

In a model of cardiac arrest, pigs were subjected to 8 min of untreated cardiac arrest and 5 min of CPR. After ROSC, animals that received ventilation with a mixture of 70% argon-30% oxygen showed a trend toward a better post resuscitation arterial pressure and myocardial ejection fraction recovery. More importantly, animals ventilated with argon achieved a fast and complete neurological recovery, in contrast to those ventilated with nitrogen and oxygen. Histopathology showed lesser brain injury in animals that were treated with argon compared to control ones. In addition, a trend towards a smaller myocardial left ventricle infarct size was observed after inhalation of argon. This histological finding was confirmed by a three-fold lower post resuscitation increase in plasmatic cardiac troponin T. Finally, ventilation with argon did not impair either respiratory exchange or arterial blood gases (*Ristagno 2014).*

*In addiction to neuroprotective effects, argon* has been reported to provide *cardiac protection. In particular*, it has been reported that argon, together with other noble gases without anesthetic properties, i.e. helium and neon, was cardioprotective when used as pre-treatment in rabbits subjected to a 30 min left anterior descending coronary artery occlusion (LAD). In that instance, animals underwent three 5-min cycles of ventilation with 70% argon in oxygen prior to LAD occlusion, as a preconditioning treatment. Argon reduced myocardial infarct size by 22% in comparison to control ventilation (*Pagel 2007).* The administration of argon as a therapeutic treatment rather than as a preventive pre-treatment in myocardial protection is not disclosed in the prior art..

### Description of the invention

It has now been found that the administration of argon through the airways concomitantly with cardiopulmonary resuscitation techniques to a subject undergoing cardiac arrest effectively protects both myocardial and neuronal cells from injuries induced by ischemia/reperfusion.

The invention accordingly concerns argon for use in the prevention of both cardiac and cerebral injury induced by ischemia/reperfusion following cardiac arrest, wherein argon or mixtures thereof are administered during cardiopulmonary resuscitation.

The concomitant administration of argon together with the standard cardiopulmonary resuscitation maneuvers allows for an improved cardioprotection combined with neuroprotection in comparison with the argon administration only after resuscitation has been achieved.

According to the invention, argon may be used in admixture with oxygen or with other inert gases. The use of an argon-oxygen mixture wherein the argon concentration ranges from 20 to 80 % by volume is preferred.

Cardiopulmonary resuscitation includes manual or mechanical chest compressions and ventilations or chest compressions only.

Argon may be administered by means of positive pressure ventilation, for instance by means of bag valve ventilation or mechanical ventilators.

Argon may also be administered passively, when a flow of the gas is put into the airways through a tubing. Examples are gas flow into a mask or nose tubing, directly into a supraglottic device or an endotracheal tube, jet ventilation, and oscillation ventilation.

Administration of the argon can be done through a closed or an open ventilation circuit.

Argon is administered to the airways as stated above in order to flow into the alveoli's and thereafter diffuse over to the blood circulation and thereafter to the cells that will be protected from injury/damage caused by cardiac arrest.

Administration of argon to the patient is performed over a time interval ranging from minutes to several hours.

The invention will be further illustrated by the following examples, reporting experimental studies showing the efficacy of argon in animal models predictive of clinical utility in humans.

### Example 1

In a severe model of cardiac arrest and CPR in the pig, treatment with inhaled argon allowed for concurrent cardiac and cerebral protection. After 12 min of untreated cardiac arrest and 5 min of CPR, animals were subjected to 4 hr ventilation with (a) 70% argon - 30% 02 or (b) 70% N2 - 30% 02. Animals receiving argon showed a significantly lower heart rate and higher mean arterial pressure and stroke volume compared to controls during the 4 hr of observation. Animals treated with argon presented also a significantly better recovery of systolic myocardial function, evaluated echocardiographically at 96 hr compared to controls. Animals treated with argon presented a significantly better neurological recovery (Cerebral Performance Category 1.7 ± 1.3) in contrast to animals in the control group (3.4 ± 1.6). Significantly lower circulating levels of hs-cTnT (median: 1332 ng/mL vs. 8015 ng/mL, p < 0.05) and NSE (median 8.5 vs. 21.2 ng/mL, p not significant) were observed in the animals ventilated with argon compared to controls. Finally, myocardial infarct size was significantly lower after argon treatment, compared to controls.

### Example 2

In a model of cardiac arrest and CPR in the rat, treatment with inhaled argon was initiated early, concurrently with chest compression prior to resuscitation. After 7 min of untreated cardiac arrest, 5 min of CPR were initiated and animals were subjected to ventilation with (a) 70% argon - 30% 02 or (b) 70% N2 - 30% 02. The above ventilation strategies were continued up to 2 hrs following resuscitation. Ventilation with argon during CPR did not impair resuscitation rate (93% resuscitation in the argon group vs. 85% in the control one). Animals receiving argon showed a higher post resuscitation mean arterial pressure and coronary perfusion pressure compared to controls. A trend toward better left ventricle ejection fraction and a lower troponin T release was also reported after ventilation with argon compared to the control ventilation. Rats ventilated with argon achieved a significantly faster and better neurological recovery compared to those ventilated with nitrogen and oxygen. Cerebral microdialysis showed a lower lactate/pyruvate ratio in the hippocampus of rats treated with argon compared to controls, indicating a lesser tissue hypoxia.

### Example 3

In a model of acute myocardial infarction by LAD occlusion in the rat, ventilation with argon reduced myocardial injury. More in details, the LAD occlusion was removed after 30 min; 5 min before reperfusion, animals were randomized to 1 hour ventilation with: (1) 70% argon - 30% oxygen; or (2) 70% nitrogen - 30% oxygen. Rats treated with argon showed significantly lower plasma level of myocardial troponin T compared to controls after 6 hours from the induction of the ischemia/reperfusion injury (3128 ng/l in argon animals vs. 7432 ng/l, p < 0.05.). Moreover, a reduced inflammatory response in terms of neutrophil infiltration in the argon ventilation group compared to control group was observed (72 cells/mm² vs. 92 cells/mm²).

### Literature

- Adrie C, Adib-Conquy M, Laurent I, et al. Successful cardiopulmonary resuscitation after cardiac arrest as a "sepsis-like" syndrome. Circulation 2002;106:562-8
- Brain Resuscitation Clinical Trial I Study Group. A randomized clinical study of thiopental loading in comatose survivors of cardiac arrest. N Engl J Med 1986;314:397-403
- Broad KD, Fierens I, Fleiss B, et al. Inhaled 45-50% argon augments hypothermic brain protection in a piglet model of perinatal asphyxia. Neurobiol Dis 2016;87:29-38
- Brücken A, Cizen A, Fera C, et al. Argon reduces neurohistopathological damage and preserves functional recovery after cardiac arrest in rats. Br J Anaesth 2013;110 Suppl 1:i106-12
- Brücken A, Kurnaz P, Bleilevens C, et al. Dose dependent neuroprotection of the noble gas argon after cardiac arrest in rats is not mediated by K(ATP)-channel opening. Resuscitation 2014;85:826-32 Brücken A, Kurnaz P, Bleilevens C, et al. Neurocrit Care 2015;22:112-20
- Coburn M, Sanders RD, Fries M, et al. Argon: The «lazy» noble gas with organprotective properties. Eur J of Anaesthesiology 2012;29:549-51
- David HN, Haelewyn B, Degoulet M, et al. Ex vivo and in vivo neuroprotection induced by argon when given after an exitotoxic or ischemic insult PLoS One 2012;7:e30934
- Fries M, Brücken A, Çizen A, et al. Combining xenon and mild therapeutic hypothermia preserves neurological function after prolonged cardiac arrest in pigs. Crit Care Med 2012;40:1297-1303
- Fries M, Nolte KW, Coburn M, et al. Xenon reduces neurohistopathological damage and improves the early neurological deficit after cardiac arrest in pigs. Crit Care Med 2008;36:2420-6
- *http:*//*www.dictionary.com*/*browse*/*righting-reflex*
- *https:*//*en.wikipedia.org*/*wiki*/*Argon*
- Jawad N, Rizvi M, Gu J, et al. Neuroprotection (and lack of neuroprotection) afforded by a series of noble gases in an in vitro model of neuronal injury. Neurosci Lett 2009;460:232-6
- Laurent I, Monchi M, Chiche JD, et al. Reversible myocardial dysfunction in survivors of out-of-hospital cardiac arrest. J Am Coll Cardiol 2002;40:2110-6
- Lippert FK, Raffay V, Georgiou M, et al. European Resuscitation Council Guidelines for Resuscitation 2010 Section 10. The ethics of resuscitation and end-of-life decisions. Resuscitation 2010;81:1445-51
- Loetscher PD, Rossaint J, Rossaint R, et al. Argon: neuroprotection in in vitro models of cerebral ischemia and traumatic brain injury. Crit Care 2009;13:R206
- Nolan JP, Neumar RW, Adrie C, et al. Post-cardiac arrest syndrome: Epidemiology, pathophysiology, treatment, and prognostication A Scientific Statement from the International Liaison Committee on Resuscitation; the American Heart Association Emergency Cardiovascular Care Committee; the Council on Cardiovascular Surgery and Anesthesia; the Council on Cardiopulmonary, Perioperative, and Critical Care; the Council on Clinical Cardiology; the Council on Stroke. Resuscitation 2008;79:350-79
- Olasveengen TM, Lund-Kordahl I, Steen PA, et al. Out-of hospital advanced life support with or without a physician: effects on quality of CPR and outcome. Resuscitation 2009;80:1248-52
- Pagel PS, Krolikowski JG, Shim YH, et al. Noble gases without anesthetic properties protect myocardium against infarction by activating pro survival signalling kinases and inhibiting mitochondrial permability transition in vivo. Anest Analg 2007;105:562-9
- Peberdy MA, Callaway CW, Neumar RW, et al. Part 9: post-cardiac arrest care: 2010 American Heart Association guidelines for cardiopulmonary resuscitation and emergency cardiovascular care. Circulation 2010;122:S768-86
- Ristagno G, Fumagalli F, Russo I, et al. Postresuscitation treatment with argon improves early neurological recovery in a porcine model of cardiac arrest. Shock 2014;41:72-8
- Ristagno G, Latini R, Vaahersalo J, et al. Early activation of the kynurenine pathway predicts early death and long-term outcome in patients resuscitated from out-of-hospital cardiac arrest. J Am Heart Assoc 2014;3(4).pii:e001094
- Ryang YM, Fahlenkamp AV, Rossaint R, et al. Neuroprotective effects of argon in an in vivo model of transient cerebral artery occlusion in rats. Crit Care Med 2011;39:1448-53
- Sandroni C, Cavallaro F, Callaway CW, et al. Predictors of poor neurological outcome in adult comatose survivors of cardiac arrest: A systematic review and meta-analysis. Part 1: Patients not treated with therapeutic hypothermia. Resuscitation 2013;84:1310-23
- Sandroni C, Cavallaro F, Callaway CW, et al. Predictors of poor neurological outcome in adult comatose survivors of cardiac arrest: A systematic review and meta-analysis. Part 2: Patients treated with therapeutic hypothermia. Resuscitation 2013;84:1324-38
- Yarin YM, Amarjargal N, Fuchs J, et al. Argon protects hypoxia-, cisplatin- and gentamycin-exposed hair cells in the newborn rat's organ of corti. Hear Res 2005;210:1-9
- Zhuang L, Yang T, Zhao H, et al. The protective profile of argon, helium, and xenon in a model of neonatal asphyxia in rats. Crit Care Med 2012;40:1724-1730.

## Claims

1. Argon for use in the prevention of both cardiac and cerebral injury induced by ischemia/reperfusion following cardiac arrest, wherein argon or mixtures thereof are administered during cardiopulmonary resuscitation.

2. Argon for use according to claim 1 wherein cardiopulmonary resuscitation includes manual or mechanical chest compressions and ventilations or chest compressions only.

3. Argon for use according to claim 1 or 2 in admixture with oxygen or with other inert gases.

4. Argon for use according to claim 3 in admixture with oxygen in a concentration of 20 to 80% by volume.

5. Argon for use according to any one of claims from 1 to 4 wherein argon is administered by means of positive pressure ventilation.

6. Argon for use according to cliam 5 wherein positive pressure ventilation is provided by means of bag valve ventilation or mechanical ventilators.

7. Argon for use according to any one of claims from 1 to 4 wherein argon is administered by means of a mask or nose tubing, directly into a supraglottic device or an endotracheal tube, jet ventilation, or oscillation ventilation.

8. Argon for use according to any one of claims from 1 to 7 wherein argon is administered through a closed or an open ventilation circuit.
